Europäisches Patentamt

European Patent Office

Office européen des brevets

(11) Numéro de publication : **0 187 090**
**B1**

(12) **FASCICULE DE BREVET EUROPÉEN**

(45) Date de publication du fascicule du brevet :
**05.04.89**

(51) Int. Cl.⁴ : **A 01 M 1/20**, A 01 M 13/00,
A 61 L 9/03

(21) Numéro de dépôt : **85402467.6**

(22) Date de dépôt : **11.12.85**

(54) Dispositif pour la destruction des insectes volants.

(30) Priorité : **21.12.84 FR 8419687**

(43) Date de publication de la demande :
**09.07.86 Bulletin 86/28**

(45) Mention de la délivrance du brevet :
**05.04.89 Bulletin 89/14**

(84) Etats contractants désignés :
**BE DE FR GB IT NL**

(56) Documents cités :
**FR--A-- 2 523 861**
**GB--A-- 1 449 448**
**GB--A-- 1 454 040**
**US--A-- 4 228 124**
**US--A-- 4 271 092**

(73) Titulaire : **SEB S.A.**
**F-21260 Selongey (FR)**

(72) Inventeur : **Seguret, René**
**1 rue du Vieux Cep Limas**
**F-69400 Villefranche/Saone (FR)**
Inventeur : **Hennuy, Jean**
**En Machon Chervinges**
**F-69400 Villefranche/Saone (FR)**

(74) Mandataire : **Bouju, André**
**Cabinet Bouju 38 avenue de la Grande Armée**
**F-75017 Paris (FR)**

EP 0 187 090 B1

## Description

La présente invention concerne un appareil comprenant un ventilateur et un dispositif pour la destruction des insectes volants, tels que les moustiques.

On connaît un dispositif comprenant une plaquette en matière absorbante imprégnée d'un produit insecticide et des moyens tels qu'une résistance électrique capables de chauffer ladite plaque à une température suffisante pour évaporer le produit insecticide dans l'atmosphère.

Ce produit insecticide se répand ainsi dans le local dans lequel est placé le dispositif et détruit les insectes qui s'y trouvent.

L'efficacité d'un tel dispositif est fonction du volume du local, des dimensions de la plaquette et de la quantité d'insecticide qui est absorbée dans cette plaquette.

Par conséquent, dans le cas d'un local de grande dimension, le dispositif n'est efficace que s'il comporte une plaquette de grande dimension imprégnée par une quantité relativement importante d'insecticide ou si plusieurs plaquettes de dimensions plus réduites sont réparties dans le local.

Par ailleurs, ces dispositifs ont une efficacité qui diminue rapidement au cours du temps.

On connaît selon l'US-A-4 228 124, un appareil comprenant un ventilateur et un dispositif pour la destruction des insectes volants qui comprend d'une part des moyens pour positionner dans le flux d'air créé par le ventilateur une plaquette en matière absorbante imprégnée d'un produit insecticide, et d'autre part des moyens de chauffage agissant sur une face de la plaquette pour chauffer la plaquette à une température suffisante pour évaporer ledit produit dans l'atmosphère par une autre face de la plaquette.

Dans toutes les formes de réalisation de ce brevet, l'air soufflé par le ventilateur est réchauffé par une résistance pour venir chauffer le produit insecticide et provoquer son dégagement.

Cette position ne peut qu'accroître la consommation du produit insecticide, au lieu de la réduire.

Le but de la présente invention est de créer un appareil pour détruire les insectes volants, présentant une efficacité et une durée d'action améliorées par rapport à celles des réalisations connues.

L'invention vise ainsi un appareil comprenant un ventilateur et un dispositif pour la destruction des insectes volants qui comprend d'une part des moyens pour positionner dans le flux d'air créé par le ventilateur une plaquette en matière absorbante imprégnée d'un produit insecticide, et d'autre part des moyens de chauffage agissant sur une face de la plaquette pour chauffer la plaquette à une température suffisante pour évaporer ledit produit dans l'atmosphère par une autre face de la plaquette.

Suivant l'invention, cet appareil est caractérisé en ce que la disposition relative du ventilateur et des moyens de chauffage est telle que le flux d'air frappe à une température ambiante ladite autre face de la plaquette.

Ainsi une face de la plaquette est chauffée par contact avec une source thermique, mais la face de dégagement est refroidie par le courant d'air, ce qui est à l'origine de l'économie de produit.

De ce fait, seule la surface de la plaquette qui est en contact avec les moyens de chauffage est portée à haute température, de sorte que le produit insecticide doit migrer à travers la plaquette vers les moyens de chauffage et n'est donc décomposé que très progressivement. Il doit ensuite retraverser la plaquette pour s'évacuer.

Les résultats d'essais effectués ont montré d'une part que la ventilation à température ambiante augmente d'une manière surprenante l'efficacité du dispositif insecticide (plaquette + moyens de chauffage) et que la plaquette insecticide a une durée d'action beaucoup plus grande que celle d'une plaquette non associée à un ventilateur, et donc a fortiori d'une plaquette chauffée par flux d'air chaud.

Selon une version avantageuse de l'invention, le ventilateur portant la plaquette est un ventilateur de faible dimension à usage domestique, utilisé normalement pour refroidir l'atmosphère ambiante.

Selon une version préférée de l'invention, la plaquette est disposée sur le socle du ventilateur du côté de l'hélice de celui-ci.

Ainsi la plaquette se trouve dans une zone où le courant d'air engendré par l'hélice propulse efficacement le produit insecticide qui s'évapore de la plaquette.

Dans le cas où les insectes volants à détruire sont des moustiques, la plaquette est de préférence imprégnée de pyrèthre, d'alléthrine, de pyréthrinoïde, de pipéronyl butoxyde ou le mélange de plusieurs de ces composés.

D'autres particularités et avantages de l'invention apparaîtront encore dans la description ci-après.

Aux dessins annexés donnés à titre d'exemples nom limitatifs :

la figure 1 est une vue en coupe longitudinale avec arrachement, d'un ventilateur équipé d'un dispositif pour la destruction des insectes, conforme à l'invention,

la figure 2 est une vue en coupe longitudinale à plus grande échelle, du dispositif conforme à l'invention,

la figure 3 est une vue en coupe suivant le plan III-III de la figure 2,

la figure 4 est une vue de dessus du dispositif.

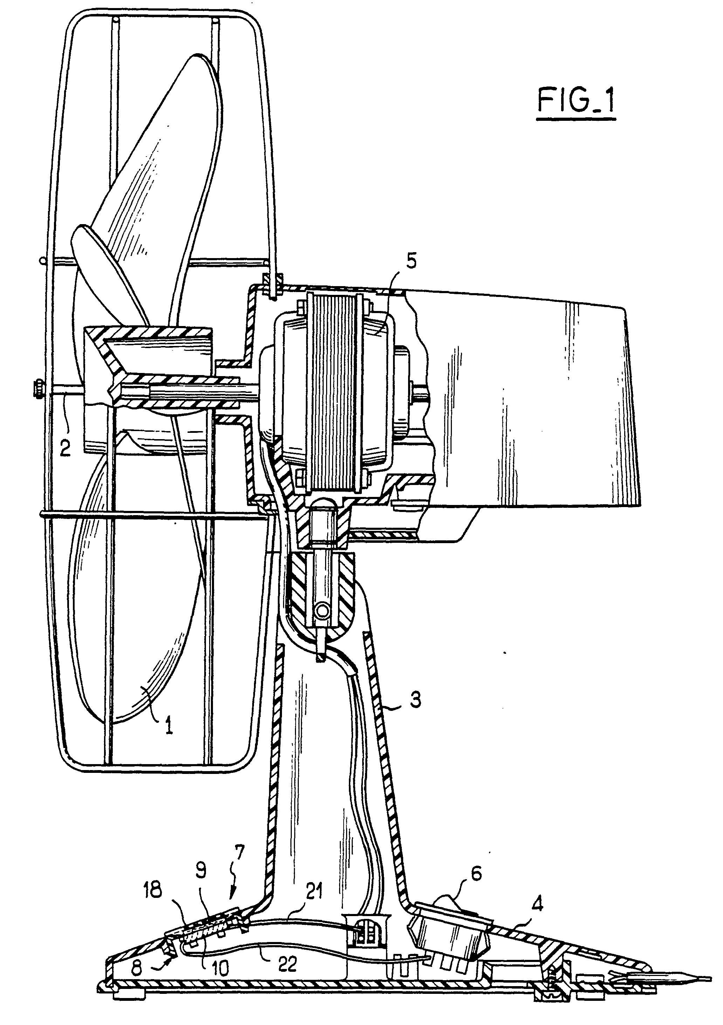

Dans la réalisation de la figure 1, le ventilateur comprend une hélice 1 montée en rotation sur un axe 2 qui est sensiblement perpendiculaire au pied vertical 3 porté par un socle 4.

L'hélice 1 est entraînée en rotation par un moteur électrique 5, dont la mise en route est commandée par un interrupteur 6.

Le socle 4 porte du côté de l'hélice 1 un dispositif 7 destiné à détruire les insectes volants, et en particulier les moustiques contenus dans le local à traiter dans lequel est placé le ventilateur.

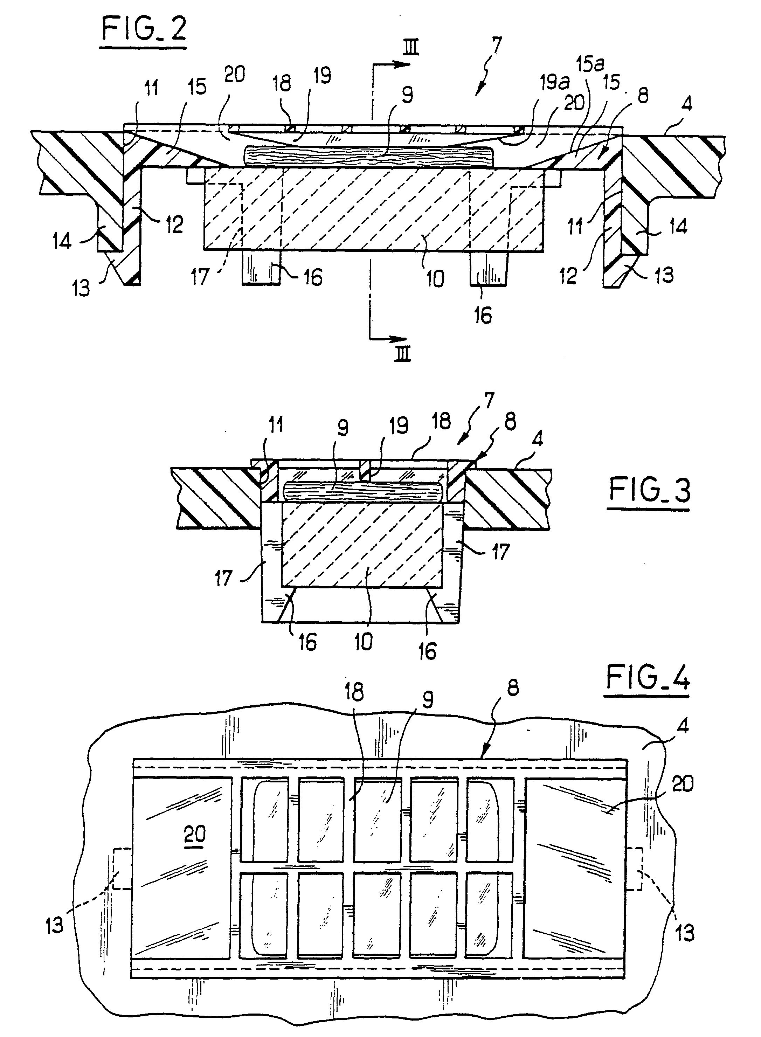

Le dispositif 7 (voir également les figures 2 à 4) comprend un support 8 en matière plastique dans lequel est disposée une plaquette 9 en matière absorbante, telle qu'un feutre en fibres non tissées imprégné par un produit insecticide tel que du pyrèthre ou de l'alléthrine. Cette plaquette 9 est appliquée sur une résistance chauffante 10, constituée dans cet exemple par une thermistance réalisée à partir d'une plaque de céramique. Dans la position représentée sur la figure 1, la plaquette 9 et la résistance 10 sont légèrement inclinées vers l'extérieur en suivant l'inclinaison du socle 4.

Le support 8 de la plaquette 9 et de la résistance 10 est encastré dans un évidement rectangulaire 11 ménagé dans la paroi du socle 4. Ce support 8 comprend (voir figure 2) à ses deux extrémités deux pattes 12 retenues par des becs d'encliquetage 13 sous des rebords 14 ménagés sous l'ouverture 11 de la paroi du socle 4.

La résistance chauffante 10 est logée entre des parois 15 du support 8 s'étendant perpendiculairement aux pattes 12 et des becs d'encliquetage 16 (voir figure 3) ménagés sur des pattes 17 perpendiculaires aux parois 15 et s'étendant de part et d'autre de la résistance 10.

Le support 8 comprend d'autre part sur sa face extérieure tournée vers l'hélice 1, une grille 18 (voir figures 2, 3 et 4) sous laquelle est placée la plaquette 9.

La plaquette 9 est appliquée contre la surface plane de la résistance 10 au moyen d'une nervure 19 en saillie sous la face intérieure de la grille 18.

Les extrémités opposées de la grille 18 comprennent chacune une ouverture 20 (voir figures 2 et 4) permettant d'introduire et de placer la plaquette 9 sous la grille 18. L'introduction de cette plaquette 9 est facilitée par des surfaces inclinées 15a et 19a ménagées sur les parois 15 et la nervure 19.

Sur la figure 1, on voit que la plaquette 9 logée dans le support 8 se trouve légèrement en arrière du plan de rotation de l'hélice 1. Cette plaquette 9 se trouve ainsi placée dans le courant d'air engendré lors de la rotation de l'hélice 1.

La résistance électrique 10 est reliée au secteur par deux conducteurs 22, 21 dont l'un 22 est branché à l'interrupteur 6, de sorte que lorsqu'on met en route le moteur 5 d'entraînement de l'hélice 1, on commande également la mise en route du chauffage de la résistance 10.

La résistance 10 est adaptée pour chauffer la plaquette 9 à une température comprise entre 50 et 100 °C et de préférence égale à 70 °C. Cette température peut être obtenue avec une puissance de chauffage inférieure à 3 watts.

Du fait de ce chauffage, le produit insecticide contenu dans la plaquette 9 s'évapore. Le courant d'air formé à proximité de la plaquette 9 chasse le produit insecticide dans le local dans lequel est placé le ventilateur, en le répartissant d'une manière sensiblement uniforme dans tout le volume de ce local et en détruisant les insectes volants se trouvant dans ce local.

Les essais ont montré que le dispositif que l'on vient de décrire était nettement plus efficace que ceux connus jusqu'à présent qui ne comportent aucun ventilateur.

Des essais comparatifs ont été réalisés dans une salle de 340 m³ contenant des cagettes renfermant des moustiques en utilisant un ventilateur à hélice de 25 cm de diamètre portant sur son socle un dispositif conforme à l'invention et en utilisant un dispositif semblable sans ventilateur.

Les résultats de ces essais ont montré qu'après deux heures de fonctionnement le taux de mortalité des moustiques atteignait 23 % dans le cas du dispositif classique et 98 % dans le cas du dispositif selon l'invention et après six heures de fonctionnement ces taux atteignaient respectivement 68 % et 100 %.

Dans le cas du dispositif conforme à l'invention, le taux de mortalité des moustiques atteignait déjà 80 % au bout d'une heure de fonctionnement du ventilateur.

Ces résultats démontrent que la ventilation augmente d'une manière surprenante l'efficacité du dispositif de destruction des moustiques.

Il a également été démontré que la plaquette 9 incorporée dans le socle d'un ventilateur avait une durée d'action beaucoup plus grande que celle d'une plaque non associée à un ventilateur.

Ainsi, il a été montré que dans le cas de la ventilation, la plaquette était efficace à 100 % jusqu'à 13 heures et qu'après 23 heures, celle-ci est encore égale à 90,6 %.

Par contre, sans ventilation, l'efficacité de la plaquette n'est plus que de 84 % après 8 heures et de 45,3 % après 13 heures.

Parallèlement, le pourcentage de moustiques « récupérant » après intoxication est plus élevé après une même durée de service lorsque la plaquette est utilisée sans ventilation, soit 48 % dans un tel cas contre 11,8 % après 13 heures de service par exemple.

Ces derniers résultats surprennent à priori. En effet, compte tenu que la dose léthale est plus rapidement atteinte dans le cas de la ventilation et que de plus, celle-ci intéresse dans un tel cas un volume d'air supérieur, il était logique de penser que la durée effective de service s'en trouverait réduite du même coup.

Une mesure de température a été faite à l'aide d'une micro-thermosonde électronique à 3 mm environ au-dessus du support de la plaquette, dans le cas de la ventilation et dans l'autre cas. Les températures trouvées ont été respectivement voisines de 36 °C et de 48,5 °C. Il est possible que les résultats précités s'expliquent par la corrélation positive qui existe certainement entre la quantité d'insecticide thermo-diffusé et la température. On est alors conduit dans un tel cas à considérer qu'en l'absence de ventilation, dans une pièce sans turbulence, il existe dans l'environnement proche de la source d'émission un taux d'insecticide nettement supérieur à celui requis pour tuer les moustiques.

Bien entendu, l'invention n'est pas limitée aux exemples que l'on vient de décrire et on peut apporter à ceux-ci de nombreuses modifications sans sortir du cadre de l'invention.

Ainsi, la plaquette 9 pourrait être placée dans un autre endroit que le socle 4 du ventilateur, pourvu que celle-ci soit exposée au courant d'air engendré par ce ventilateur et que ce courant d'air puisse entraîner avec lui le produit insecticide contenu dans la plaquette. De plus, dans le ventilateur décrit à titre d'exemple, on pourrait prévoir un interrupteur distinct de celui qui commande la mise en route du moteur pour commander le chauffage de la résistance 10 de façon à pouvoir arrêter ce chauffage à tout moment.

**Revendications**

1. Appareil comprenant un ventilateur et un dispositif pour la destruction des insectes volants qui comprend d'une part des moyens pour positionner dans le flux d'air créé par le ventilateur une plaquette (9) en matière absorbante imprégnée d'un produit insecticide, et d'autre part des moyens de chauffage (10) agissant sur une face de la plaquette pour chauffer la plaquette (9) à une température suffisante pour évaporer ledit produit dans l'atmosphère par une autre face de la plaquette, caractérisé en ce que la disposition relative du ventilateur et des moyens de chauffage est telle que le flux d'air frappe à température ambiante ladite autre face de la plaquette.

2. Appareil conforme à la revendication 1, caractérisé en ce qu'il comprend un socle (4) surmonté par un pied (3) sensiblement vertical, portant l'hélice (1) du ventilateur, montée en rotation suivant un axe sensiblement perpendiculaire au pied, et en ce que la plaquette (9) est disposée sur le socle (4) du côté de l'hélice (1).

3. Appareil conforme à la revendication 2, caractérisé en ce que la plaquette (9) est disposée sur le socle (4) entre le pied (3) et le plan de rotation de l'hélice (1).

4. Appareil conforme à l'une des revendications 1 à 3, caractérisé en ce que la plaquette (9) est imprégnée de pyrèthre, d'alléthrine, de pyréthrinoïde, de pipéronybutoxyde ou le mélange de plusieurs de ces composés.

5. Appareil conforme à l'une des revendications 1 à 4, caractérisé en ce que la plaquette (9) est en contact avec une résistance chauffante (10) capable de chauffer ladite plaquette à une température comprise entre 50 et 100°C.

6. Appareil conforme à la revendication 5, caractérisé en ce que la résistance (10) est une thermistance réalisée à partir d'une plaque de céramique.

7. Appareil conforme à l'une des revendications 2 à 6, caractérisé en ce que la plaquette (9) et la résistance (10) sont montées dans un support (8) qui est encastré dans un évidement (11) ménagé dans le socle (4) du ventilateur.

8. Appareil conforme à la revendication 7, caractérisé en ce que le support (8) comporte sur sa face extérieure tournée vers l'hélice (1) du ventilateur, une grille (18) sous laquelle est placée la plaquette (9).

9. Appareil conforme à la revendication 8, caractérisé en ce que la grille (18) comprend une ouverture (20) permettant d'introduire et de placer la plaquette (9) sous cette grille (18).

10. Appareil selon l'une des revendications 1 à 9, caractérisé en ce que le ventilateur est d'un type approprié pour la ventilation d'une pièce.

**Claims**

1. Apparatus comprising a fan and a device for the destruction of flying insects which is provided on the one hand with means for positioning in the airstream produced by the fan a plate (9) of absorbent material impregnated with an insecticide product and on the other hand with heating means (10) for producing action on one face of the plate in order to heat the plate (9) to a temperature which is sufficient to evaporate said product in the atmosphere through another face of the plate, characterized in that the relative arrangement of the fan and of the heating means is such that the airstream impinges on said other face of the plate at room temperature.

2. Apparatus in accordance with claim 1, characterized in that it comprises a base (4) surmounted by a substantially vertical foot (3) for carrying the fan impeller (1) which is rotatably mounted on an axis substantially perpendicular to the foot, and that the plate (9) is disposed on the base (4) on the same side as the impeller (1).

3. Apparatus in accordance with claim 2, characterized in that the plate (9) is disposed on the base (4) between the foot (3) and the plane of rotation of the impeller (1).

4. Apparatus in accordance with one of claims 1 to 3, characterized in that the plate (9) is impregnated with pyrethrum, allethrin, pyrethrinoid, piperonylbutoxide or a mixture of several of these compounds.

5. Apparatus in accordance with one of claims 1 to 4, characterized in that the plate (9) is in contact with a heating resistor (10) which is capable of heating said plate to a temperature comprised between 50 and 100 °C.

6. Apparatus in accordance with claim 5, characterized in that the resistor (10) is a thermistor fabricated from a ceramic plate.

7. Apparatus in accordance with one of claims 2 to 6, characterized in that the plate (9) and the resistor (10) are mounted in a support (8) which is inserted in a hollowed-out portion (11) formed in the base (4) of the fan.

8. Apparatus in accordance with claim 7, characterized in that the support (8) is provided on its external face which is directed towards the impeller (1) of the fan with a grid (18) beneath which is placed the plate (9).

9. Apparatus in accordance with claim 8, characterized in that the grid (18) has an opening (20) for introducing and placing the plate (9) beneath this grid (18).

10. Apparatus in accordance with one of claims 1 to 9, characterized in that the fan is of a suitable type for ventilation of a room.

**Patentansprüche**

1. Gerät mit einem Lüfter sowie einer Vorrichtung für die Zerstörung von fliegenden Insekten, welches einerseits Mittel zum Positionieren eines Plättchens (9) in dem von dem Lüfter erzeugten Lufstrom, welches aus einem absorbierenden, mit einem Insektizidmittel durchtränkten Stoff besteht, sowie andererseits Erwärmungsmittel (10) umfaßt, welche auf eine Fläche des Plättchens so einwirken, daß das Plättchen (9) auf eine Temperatur erwärmt wird, die hoch genug ist, damit dieses Insektizidmittel über eine andere Fläche des Plättchens in die Atmosphäre verdunstet, dadurch gekennzeichnet, daß die relative Anordnung zwischen dem Lüfter und den Erwärmungsmitteln so ist, daß bei Umgebungstemperatur der Luftstrom auf die andere Fläche des Plättchens trifft.

2. Gerät nach Anspruch 1, dadurch gekennzeichnet, daß es einen von einem im wesentlichen senkrechten Fuß (3) überragten Sockel (4) umfaßt, welcher die Schraube (1) des Lüfters trägt, die gemäß einer zu dem Fuß im wesentlichen senkrechten Achse drehbar montiert ist, und daß das Plättchen (9) auf der Seite der Schraube (1) auf dem Sockel (4) angebracht ist.

3. Gerät nach Anspruch 2, dadurch gekennzeichnet, daß das Plättchen (9) zwischen dem Fuß (3) und der Drehebene der Schraube (1) auf dem Sockel (4) angebracht ist.

4. Gerät nach einem der Ansprüche 1 bis 3, dadurch gekennzeichnet, daß das Plättchen (9) mit Pyrethrum, Allethrin, Pyrethrinoid, Piperonyl-Butoxid bzw. mit einer Mischung aus mehreren dieser Verbindungen durchtränkt ist.

5. Gerät nach einem der Ansprüche 1 bis 4, dadurch gekennzeichnet, daß das Plättchen (9) mit einem Heizwiderstand (10) in Berührung steht, welcher in der Lage ist, dieses Plättchen auf eine Temperatur zwischen 50 und 100 °C zu erwärmen.

6. Gerät nach Anspruch 5, dadurch gekennzeichnet, daß der Widerstand (10) ein aus einer Keramikplatte gebildeter Thermistor ist.

7. Gerät nach einem der Ansprüche 2 bis 6, dadurch gekennzeichnet, daß das Plättchen (9) sowie der Widerstand (10) in einem Träger (8) montiert sind, der in einer in dem Sockel (4) des Lüfters eingerichteten Aussparung (11) eingefügt ist.

8. Gerät nach Anspruch 7, dadurch gekennzeichnet, daß die der Schraube (1) des Lüfters zugewandte Außenfläche des Trägers (8) ein Gitter (18) umfaßt, unter welchem das Plättchen (9) angebracht ist.

9. Gerät nach Anspruch 8, dadurch gekennzeichnet, daß das Gitter (18) eine Öffnung (20) umfaßt, welche die Einführung sowie die Anbringung des Plättchens (9) unter diesem Gitter (18) ermöglicht.

10. Gerät nach einem der Ansprüche 1 bis 9, dadurch gekennzeichnet, daß der Lüfter für die Belüftung eines Raumes geeignet ist.

FIG_1

## FIG_2

## FIG_3

## FIG_4